# EUROPÄISCHE PATENTSCHRIFT

(11) **EP 0 327 693 B1**
(45) Veröffentlichungstag und Bekanntmachung des Hinweises auf die Patenterteilung: **15.09.1993**
(21) Anmeldenummer: 88119755.2
(22) Anmeldetag: 26.11.1988
(51) Int. Cl.: A61B 3/16, A61B 3/12, A61B 5/02

(54) **Ophthalmologisches Gerät**
Ophthalmic device
Dispositif ophtalmique

(30) Priorität: 09.02.1988 CH 466/88
(43) Veröffentlichungstag der Anmeldung: 16.08.1989
(73) Patentinhaber: Robert, Yves, Prof. Dr. med., 8044 Zürich (CH)
(72) Erfinder: Robert, Yves, Prof. Dr. med., 8044 Zürich (CH)
(74) Vertreter: Keller, René, Dr.

(56) Entgegenhaltungen:
- DE-A- 3 032 164
- DE-A- 3 421 701
- TRANSACTIONS ON BIOMEDICAL ENGINEERING Band BME-29, Nr. 3, März 1982, Seiten 178-183, New York, USA; R.C. ZEIMER u.a.: "An Instrument for Self-Measurement of Intraocular Pressure"

## Beschreibung

Die Erfindung betrifft ein ophthalmologisches Gerät gemäß dem Oberbegriff des Patentanspruchs 1.

Optische Bauelemente (abbildendes optisches Bauelement) der abbildenden (geometrischen) Optik sind Bauelemente, welche einfallende optische Strahlen gemäß den Gesetzen der geometrischen Optik wieder als optische Strahlen mit oder ohne Richtungsänderung gegenüber den einfallenden Strahlen aussenden. Lichtleiterbündel sind keine optische Bauelemente der abbildenden (geometrischen) Optik, da jeder Lichtleiter des Bündels nur den Leuchtdichtemittelwert der auf ihn fallenden Strahlung überträgt.

Ein gattungsgemäßes ophthalmologisches Gerät wird mit einem Andruckkörper als Funduskontaktglas nach Goldmann weltweit in der augenärztlichen Praxis verwendet, um z. B. mit Hilfe einer Spaltlampe, einem Kondensorsystem und einem teildurchlässigen Spiegel den Augenhintergrund zu beleuchten und über eine Betrachtungsoptik zu untersuchen. Das Kontaktglas hat eine der Hornhautwölbung angepaßte konkave Fläche und verringert hierdurch Reflexionen durch die Beleuchtung an der Hornhautoberfläche, bzw. Kontaktglasoberfläche. Mit der dieser angepaßten Fläche gegenüberliegenden Fläche bildet das Kontaktglas als optisches Bauelement eine Linse im gesamten Beleuchtungs- und Beobachtungssystem. Das Funduskontaktglas ist ein optisches Bauelement der abbildenden (geometrischen) Optik.

Eine Druckmessung des Augeninnendrucks ist heute u. a. mittels eines Applanationstonometers (nach dem Gesetz von Imbert-Fick, 1885) möglich, bei dem ein durchsichtiger Druckkörper mit einer ebenen Auflagefläche derart an die gewölbte Hornhaut gedrückt wird, daß diese auf einer Fläche von annähernd 7 mm² abgeplattet wird. Vor der Messung wird ein Streifen Fluoreszeinpapier in den Bindehautsack eingelegt. Während der Messung wird das Auge durch eine Spaltlampe mit Blaufilter beleuchtet. Im Bereich der Berührungsfläche von Hornhaut und Druckkörper wird der im blauen Licht grüngelblich leuchtende fluoreszeinhaltige Tränenfilm verdrängt, so daß die Grenze zwischen abgeplatteter und gewölbter Hornhaut gut erkennbar ist. Die zur Abplattung notwendige Andruckkraft ist ein Maß des Augeninnendrucks.

Ein nicht gattungsgemäßes ophthalmologisches Gerät als sog. Applanationstonometer ist aus der DE-A 34 21 701 bekannt. Dieses Applanationstonometer dient zur Messung des Augeninnendrucks mittels eines axial bewegbaren, aus einer Vielzahl von Lichtleitern bestehenden Meßelements. Das Lichtleiterbündel steht senkrecht auf der Applanationsfläche auf der Hornhaut des Auges. In das der Applanationsfläche abgewandte Ende des Bündels wird Licht eingekoppelt. Im Bereich der Applanationsfläche, wie auf Seite 33, Zeile 18 ff ausgeführt, wird das Licht von der Oberfläche der Hornhaut ungebrochen reflektiert und läuft durch den gleichen oder einen benachbarten Lichtleiter als Reflexionslicht R zurück. Im Bereich der Tränenflüssigkeit wird das Licht ebenfalls erst auf der Hornhaut reflektiert, und zwar unter einem Winkel. Wenn die Reflexe dieses Lichts als gebrochenes Reflexionslicht R' in einen Lichtleiter gelangen, ist deren Lichtstärke durch die Absorption in der Tränenflüssigkeit und die durch das schräge Auftreffen auf den Lichtleiter verringerte Leuchtdichte schwächer als die Lichtstärke des Reflexionslichts R. Die den zur Applanationsfläche abgewandten Enden der Lichtleiter nachgeschalteten Detektoren sind derart ausgelegt, daß sie die übertragene abgeschwächte Lichtstärke nicht auswerten.

Mit dem Lichtleiterbündel der DE-A 34 21 701 kann nur eine "gerasterte" Leuchtdichteverteilung der unmittelbar auf dem Bündelende anliegenden Hornhaut sowie der vom Bündelende geringfügig distanzierten Hornhautstellen, welche über die lichtleitende Tränenflüssigkeit mit den Bündelende verbunden sind, übertragen werden. Eine Betrachtung des Augenhintergrunds ist mit der in der DE-A 34 21 701 beschriebenen Vorrichtung nicht möglich.

Bis heute konnten u. a. folgende Untersuchungen gar nicht oder auch bei Mitwirkung eines Assistenten nur umständlich und/oder ungenau durchgeführt werden:
druckkörper (1) in seine Zum Auge (6) zentrierte Lage zu verschieben ist.
- Messung des pulsierenden Augeninnendrucks, um Aufschlüsse über gewisse Krankheiten der Hirngefäße zu erhalten.
- Tonographie, bei der der Augeninnendruck durch einen seitlich an das Auge angesetzten Saugnapf künstlich erhöht, der Saugnapf entfernt, und dann der Augeninnendruck in kurzen Zeitabständen wiederholt zu messen wäre. Die Methode der Tonographie ist heute in Vergessenheit geraten, da sie mit den bis jetzt zur Verfügung stehenden Geräten zu aufwendig und zu ungenau ist. Ihr theoretischer Wert ist jedoch unbestritten.
- Ophthalmodynamometrie, bei der mit dem oben erwähnten Saugnapf der Augendruck kräftig erhöht wird, um durch die Pulsaktionen der Netzhautgefäße am Augenhintergrund unter gleichzeitiger Messung des Augendruckes den Blutdruck der Hirngefäße zu bestimmen. Für die Erkennung von Hirngefäßstörungen ist diese Messung von eminenter Bedeutung. Sie konnte bis jetzt nur indirekt und nur von zwei Personen an Kliniken durchgeführt werden.
- Photopapillometrie (EP-A 136440), bei der der Augeninnendruck nur geringfügig erhöht, und mittels eines Photopapillometers (Reflexionsmeßgerät) die Helligkeit des Sehnervs (blinder Fleck) und dessen Änderung unter künstlicher Druckerhöhung bestimmt wird. Hierdurch ist eine Aussage über die Entstehung oder den Verlauf des grünen Stars möglich.

Um Meßfehler zu vermeiden, muß über der Hornhautmitte des Auges tonometriert (gemessen) werden. Dabei konnte bisher nicht gleichzeitig der Augenhintergrund beobachtet werden. Man konnte entweder den Augenhintergrund betrachten oder den Druck messen, aber nicht beides gleichzeitig.

Die Erfindung löst die Aufgabe, während der Betrachtung des Augenhintergrundes gleichzeitig den Augeninnendruck zu messen mit den im kennzeichnenden Teil von Anspruch 1 angegebenen Merkmalen.

Die durch die Erfindung erzielten Vorteile sind im wesentlichen darin zu sehen, daß einige Augenuntersuchungen, wie die u. a. oben erwähnten, ohne größeren apparativen und personellen Aufwand, stark vereinfacht und von nur einer einzigen Person ohne zusätzliche Hilfsmittel, und daß andere Untersuchungen, wie z. B. die Tonographie erst durch das erfindungsgemäße ophthalmologische Gerät mit der erforderlichen Genauigkeit durchgeführt werden können.

Die Erfindung wird im folgenden anhand der beiliegenden, lediglich beispielsweise Ausführungswege darstellenden Zeichnungen näher erläutert. Es zeigen:
- Fig. 1: Prinzipskizze des optischen Strahlenganges mit einem Längsschnitt durch ein erfindungsgemäßes ophthalmologisches Gerät,
- Fig. 2: eine Draufsicht auf den Andruckkörper des Geräts von Fig. 1,
- Fig. 3: einen Längsschnitt durch eine Variante des Andruckkörpers,
- Fig. 4: einen Längsschnitt durch eine zweite Variante des Andruckkörpers.

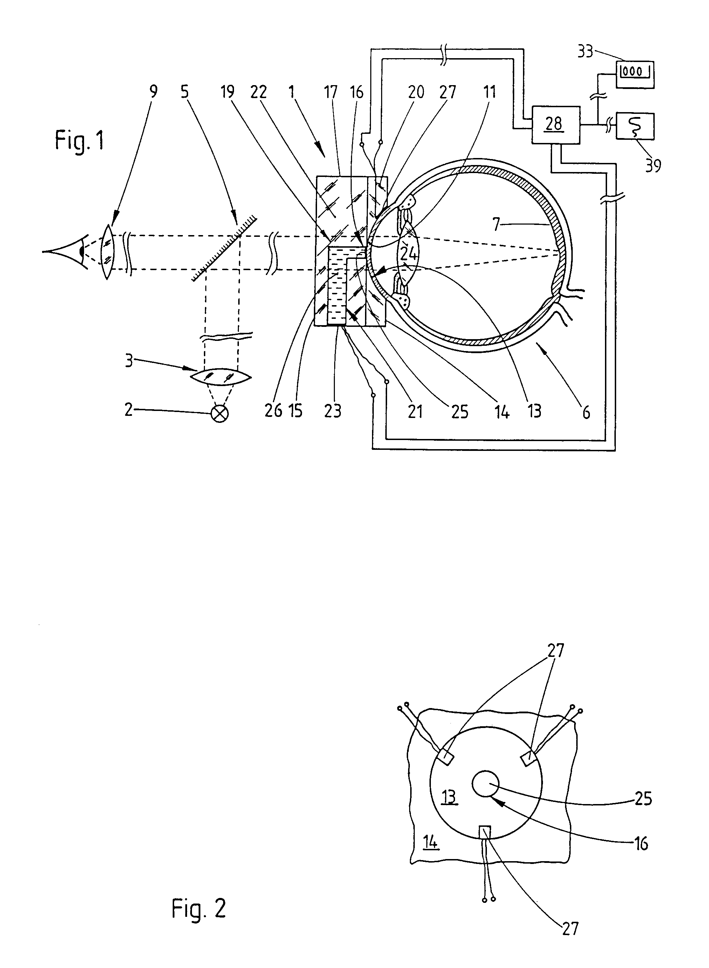

In Fig. 1 wird der Augenhintergrund 7 durch einen annähernd zylinderförmigen Andruckkörper 1 hindurch mit Hilfe einer Spaltlampe 2, einem Kondensorsystem 3 und einem teildurchlässigen Spiegel 5 beleuchtet, und über eine Betrachtungsoptik 9 untersucht. Der Zweck dieses Andruckkörpers 1 ist es, die Lichtreflexion an der Oberfläche der Hornhaut 11 weitgehend zu eliminieren. Die dem Auge 6 zugewandte Oberfläche 13 des Andruckkörpers 1 ist rotationssymmetrisch konkav und im Kontaktbereich mit der Hornhaut 11 in ihrer Krümmung der Krümmung der Hornhaut 11 des Auges 6 angepaßt. Außerhalb des Kontaktbereichs geht die Oberfläche 13 in eine ebene Zylindergrundfläche 14 über. Die der Fläche 13 gegenüberliegende Fläche 15 ist eben, mit einer Antireflexionsschicht versehen, und liegt senkrecht zur Symmetrieachse der Fläche 13; sie kann aber auch eine sphärische oder asphärische rotationssymmetrische Fläche sein. Die Fläche 15 ist an die optischen Anforderungen für Beleuchtung und Betrachtung des Augenhintergrundes 7 angepaßt. An die seitliche Begrenzung des Andruckkörpers 1, die bevorzugt als eine Zylindermantelfläche 17 ausgebildet wird, werden keine optischen Anforderungen gestellt.

In der Mitte der Fläche 13 befindet sich eine Öffnung 16 eines Hohlraums 19, der sich abgewinkelt als Kanal 21 zur Zylindermantelfäche 17 erstreckt, an der er durch einen Drucksensor 23 flüssigkeitsdicht abgeschlossen ist. Der Hohlraum 19 und der Kanal 21 sind mit einer Flüssigkeit 26, die mindestens annähernd gleichen Brechungsindex wie das Material des Andruckkörper 1 hat, vollständig und gasblasenfrei gefüllt.

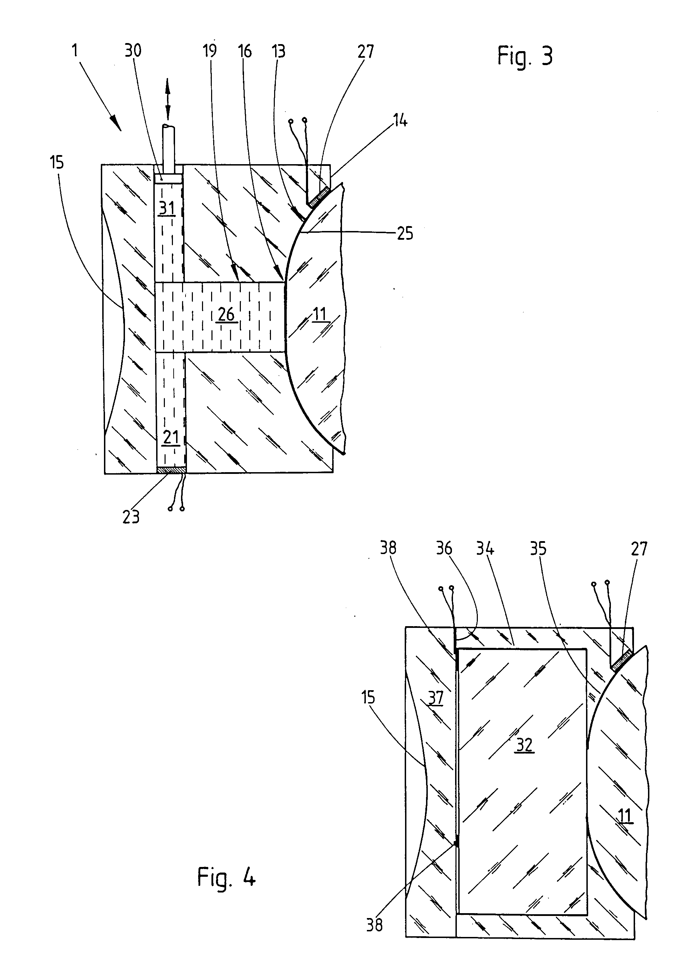

Die Öffnung 16 des Hohlraums 19 ist an der der Hornhaut 11 zugewandten Seite mit einer durchsichtigen Membran 25 (vergleiche auch Fig. 3) abgeschlossen. Die mit der Membran 25 verschlossene Öffnung 16 hat vorzugsweise einen Durchmesser von 3 mm (entsprechend einer Meßfläche von 7 mm² ).

Am Rand der Oberfläche 13 außerhalb des optisch genutzten Bereiches liegen mit gleichem Winkelabstand zueinander drei Drucksensoren 27 (Fig. 2) zur Messung des Anpreßdrucks des Andruckkörpers 1 an das Auge 6.

Nach Fig. 1 ist der Andruckkörper 1 zur einfacheren Montage der Membran 25 aus zwei zylindrischen Teilen 20 und 22 zusammengesetzt, wobei die Flächen 13 und 14 zum Teil 20, und die Fläche 15 zum Teil 22 gehören. Die Membran 25 liegt zwischen den ebenen Grenzflächen dieser Teile 20, 22. Beide Grenzflächen sind mit einem Klebstoff, der mindestens annähernd gleichen Brechungsindex wie die beiden zylindrischen Teile 20, 22 hat, zusammengeklebt.

Bei einer Augenuntersuchung wird der Andruckkörper 1 gegen die Hornhaut 11 gedrückt, und zwar so, daß die mit der Membran 25 verschlossene Öffnung 16 auf der Mitte der Hornhaut 11 sitzt. Der Hohlraum 19 ist vollständig mit Flüssigkeit 26 gefüllt und hermetisch abgeschlossen. Da die Membran 25 auf der Öffnung 16 ihre ebene Oberfläche während des Andrückens beibehält, wird die Hornhat 11 abgeplattet (Applation). Der Augeninnendruck drückt nun über die Hornhaut 11 auf die Membran 25, und diese wiederum auf die Flüssigkeit 26 im Hohlraum 19. Da jede Flüssigkeit 26 inkompressibel ist, und auch der Drucksensor 23 keine bemerkenswerte Volumenänderung ausführt, ist der Druck der Flüssigkeit 26 auf die Wände des Hohlraums 19 und auf den Drucksensor 23 gleich dem Augeninnendruck, mit der bekannten Einschränkung, daß letzterer durch die Applation um etwa 2% erhöht worden ist.

Der einzige relevante Meßfehler, der noch entstehen kann, ist durch das Anpressen des Andruckkörpers 1 an das Auge 6 bedingt. Unterschiedlich starkes Anpressen führt zu unterschiedlich starker künstlicher Augeninnendruckerhöhung. Dieser Fehler wird wie folgt eliminiert. Der Anpreßdruck wird durch die drei Drucksensoren 27 gemessen und die Differenzen der Meßwerte durch eine elektronische Auswerteschaltung 28 ermittelt. Bei hinreichend übereinstimmenden Meßwerten wird ein Mittelwert aus den drei Meßwerten gebildet und dieser mit dem Meßwert des Drucksensors 23 zur automatischen Berechnung des tatsächlichen Augeninnendrucks verwendet und durch eine Anzeigeanordnung 33 angezeigt. Durch Anschluß eines Schreibers 39 an die elektronische Schaltung 28 läßt sich der zeitliche Druckverlauf darstellen.

Bei unterschiedlichen Meßwerten der drei Drucksensoren 27 wird die Richtung bzw. die Koordinatenrichtung bestimmt, in der der Andruckkörper 1 zu verschieben ist.

Die Beleuchtung des Augenhintergrundes 7 erfolgt, wie in Fig. 1 schematisch gezeigt, von der Lampe 2 über das Kondensorsystem 3, den teildurchlässigen Spiegel 5, den Andruckkörper 1, durch die Hornhaut 11 und die Augenlinse 24. Die Untersuchung erfolgt über die Beobachtungsoptik 9, ebenfalls über den teildurchlässigen Spiegel 5 und dann analog zum Beleuchtungsstrahlengang. Da der Andruckkörper 1 als abbildendes optisches Bauelement ausgeführt ist, und sowohl der Drucksensor 23 als auch die drei Drucksensoren 27 außerhalb des Strahlenganges liegen, ergibt sich keine Beeinträchtigung von Beleuchtung und Beobachtung.

Zur genauen Bestimmung des Augendruckes soll die mit der Membran 25 abgedeckte Öffnung 16 tangential in der Mitte der Hornhaut 11 andrücken. Der vollständig mit Flüssigkeit 26 gefüllte Hohlraum 19 ist einwandfrei durch die Membran 25 verschlossen. Die einwandfreie Druckübertragung von Augeninnendruck über die Hornhaut 11, die an ihr anliegende Membran 25 und durch die Flüssigkeit 26 zum Drucksensor 23 erfolgt nur, sofern keine Gasblasen in der Flüssigkeit 26 vorhanden sind. Wie oben beschrieben, kann die Membran 25 zwischen den zwei Teilen 20 und 22 des Andruckkörpers 1 gespannt und befestigt sein, oder sie kann direkt auf die gekrümmte Fläche 13 geklebt werden. Die letztere Variante stellt an die Herstellung größere Anforderungen, da sonst die Gefahr besteht, daß die angeklebte Folie sich entlang der Öffnung 16 löst.

Die Membran 25 kann aber auch nur zwischen die beiden Teile 20 und 22 gelegt, und die beiden Teile 20 und 22 mit einem nicht gezeichneten Spannmechanismus zusammengespannt werden. Diese Ausführungsart hat den Vorteil, daß sie zur Desinfektion leicht auseinandergenommen werden kann.

In den Hohlraum 19 kann, wie in Fig. 3 dargestellt, eine mit einem Kolben 30 als Verdrängungskörper verschlossene Zylinderbohrung 31 von der Seitenfläche 17 aus hineinführen. Durch Verschieben dieses Kolbens 30 kann dann die Planizität, bzw. die Wölbung der Membran 25 über der Öffnung 16 eingestellt werden. Die Zylinderbohrung 31 mit dem Kolben 30 erleichtert die Montage der Membran 25, da nach der Montage noch Korrekturmöglichkeiten für die Membranwölbung bestehen. Die Zylinderbohrung 31 mit dem Kolben 30 kann auch bei dem oben beschriebenen zweiteiligen Andruckkörper 20, 22 (Fig. 1) als Variante verwendet werden.

Der Sensor 23 sitzt wegen der einfachen Montage bevorzugt an der Zylinderwand 17, kann aber an jeder beliebigen Stelle der Wand des Hohlraums 19 außerhalb des Beleuchtungs- und Untersuchungsstrahlenbündels sitzen.

Der mit der Membran 25 abgeschlossene und mit der Flussigkeit 26 zur Druckübertragung auf den Drucksensor 23 gefüllte Hohlraums 19, kann dahingehend geändert werden, daß ein durchsichtiger zylindrischer Körper 32 (Fig. 4) mit ausreichender optischer Homogenität mit Spielpassung in ihn eingepaßt wird. In diesem Fall hat der zylinderförmige Andruckkörper ebenfalls eine der Fläche 13 analoge konkave Fläche 35. Der Hohlraum ist zylinderförmig und sein Durchmesser so groß, daß die begrenzenden Wände außerhalb des Strahlenbündels liegen. Eine der gekrümmten Fläche 35 gegenüberliegende Kreisringfläche 36 wird mit einer ebenen Scheibe 37 mit ausreichender optischer Qualität abgeschlossen. Die Scheibe 37 trägt an ihrem optisch nicht genutzten Rand innerhalb des Kreisringes in gleichem Abstand drei gleiche Drucksensoren 38, die in ihrer Wirkungsweise dem Drucksensor 23 ähnlich sind. Die Hornhaut 11 drückt jetzt direkt auf den Körper 32 und dieser auf die Drucksensoren. Zur Vermeidung störender Lichtreflexe tragen die Oberfächen des Körpers 32 und der Scheibe 37 bevorzugt eine Antireflexionsbeschichtung.

In Abänderung kann der durchsichtige Körper 32 auch als Teil eines Hebelarms ausgebildet sein (nicht dargestellt), und die Druckmessung mit einem Drucksensor in der Nähe des Drehpunktes des Hebelarms zur Erhöhung der Meßempfindlichkeit vorgenommen werden. Bei dieser Konstruktion sollte darauf geachtet werden, daß der Weg des Hebelarms beim Andrücken der Meßplatte an die Hornhaut des Auges verschwindend klein ist. Auch hier muß zur Vermeidung störenden Reflexionen die Vorder- und Rückseite der Meßplatte entspiegelt sein.

## Patentansprüche

1. Ophthalmologisches Gerät mit einem an die Hornhaut **(11)** des Auges **(6)** zu drückenden, als optisches Bauelement der abbildenden Optik ausgebildeten Andruckkörper **(1)**, durch den der Augenhintergrund **(7)** beleucht- und beobachtbar ist, **gekennzeichnet durch** eine Druckmeßvorrichtung **(19, 21, 23, 25, 26, 27)** zur Bestimmung des Augeninnendrucks, die mindestens ein Druckaufnahmeelement (25, 32) und/oder einen Drucksensor (27) aufweist, das Bzw. der an der der Hornhaut (11) des Auges (6) anzudrückenden Oberfläche (13) des Andruckkörpers (1) angeordnet ist, so daß damit der Augeninnendruck bestimmbar sowie gleichzeitig der Augenhintergrund beobachtbar ist.

2. Ophthalmologisches Gerät nach Anspruch 1, dadurch gekennzeichnet, daß das Druckaufnahmeelement (25, 32) durchsichtig ist und im mittleren Bereich der an die Augenhornhaut (11) anzudrückenden Oberfläche (13) des Andruckkörpers (1) angeordnet ist.

3. Opthalmologisches Gerät nach Anspruch 1 oder 2, bei dem die an die Augenhornhaut (11) anzudrückende Oberfläche (13) des Andruckkörpers (1) eine dem Auge (6) annähernd angepaßte konkave Form hat, dadurch gekennzeichnet, daß die an die Hornhaut (11) anzulegende Fläche des Druckaufnahmeelement (25, 32) eben ist, so daß sie tangential an die Hornhaut (11) andrückbar ist.

4. Opthalmologisches Gerät nach einem der Ansprüche 1 bis 3, dadurch gekennzeichnet, daß das Druckaufnahmeelement (25, 32) einen vollständig mit einer durchsichtigen Flüssigkeit (26) gefüllten Hohlraum (19) im Andruckkörper (1) abschließt, wobei der Brechungsindex der Flüssigkeit (26) wenigstens annähernd gleich dem des Andruckkörpers (1) ist, und daß die Flüssigkeit (26) einen ersten Drucksensor (23) beaufschlagt.

5. Ophthalmologisches Gerät nach Anspruch 4, dadurch gekennzeichnet, daß an seinem optisch nicht genutzen Rand ein verschiebbarer Verdrängungkörper (30) in den mit Flüssigkeit (26) gefüllten Hohlraum (19, 31) hineinragt.

6. Opthalmologisches Gerät nach einem der Ansprüche 1 bis 3, dadurch gekennzeichnet, daß das Druckaufnahmeelement (32) direkt auf mindestens einen Drucksensor (38) wirkt.

7. Opthalmologisches Gerät nach Anspruch 4 bis 6, dadurch gekennzeichnet, daß der Drucksensor (23) am optisch nicht genutzten Rand des Andruckkörpers (1) angeordnet ist.

8. Ophthalmologisches Gerät nach Anspruch 4, dadurch gekennzeichnet daß das Druckaufnahmeelement (25) eine den Hohlraum (19) flüssigkeitsdicht abschließende, durchsichtige Membran (25) ist.

9. Opthalmologisches Gerät nach einem der Ansprüche 4 bis 8, bei dem die an die Augenhornhaut (11) anzudrückende Oberfläche (13) des Andruckkörpers (1) eine dem Auge (6) annähernd angepaßte konkave Form hat, dadurch gekennzeichnet, daß mindestens ein zweiter Drucksensor (27) am optisch nicht genutzten Rand in der konkaven Fläche (13) zur Messung des Anpreßdruckes des Andruckkörpers (1) an das Auge (6) angeordnet ist, und daß die Druckmeßvorrichtung (19 21, 23, 25, 26, 27) eine elektronische Auswerteschaltung (28) hat, welche die Differenz der elektrischen Signale des ersten (23) und des zweiten (27) Sensors, oder die Differenz zwischen dem Signal des ersten Sensors (23) und dem Mittelwert der Signale der zweiten Sensoren (27) errechnet, wobei eine diese Differenz direkt und/oder den aus ihr ermittelten Augendruck anzeigende Anzeigevorrichtung (33, 39) vorgesehen ist.

10. Ophthalmologisches Gerät nach Anspruch 9, dadurch gekennzeichnet, daß mindestens drei zweite Sensoren (27) mit wenigstens annähernd gleichen Winkelabständen voneinander am Rand in der konkaven Fläche (13) angeordnet sind, und daß die Anzeigevorrichtung (33, 39) mit elektronischer Auswerteschaltung (28) bei hinreichend miteinander übereinstimmenden Signalen dieser drei Drucksensoren (27), die in bezug auf das Auge (6) zentrierte Lage des Andruckkörpers (1), und bei unterschiedlichen Signalen dieser drei Sensoren (27), die Richtung oder Koordinatenrichtungen anzeigt, in der bzw. denen der Andruckkörper (1) in seine zum Auge (6) zentrierte Lage zu verschieben ist.

## Claims

1. An ophthalmological appliance with a contact pressure body (1) for pressing against the cornea (11) of the eye (6) and constructed as an optical component of the imaging optics, by means of which the retina (7) is illuminated and can be observed, characterised by a pressure measuring device (19, 21, 23, 25, 26, 27) for determining the internal pressure of the eye, which has at least one pressure absorption element (25, 32) and/or one pressure sensor (27) which is disposed at the surface (13) of the contact pressure body (1) which presses against the cornea (11) of the eye (6), so that the internal pressure of the eye can thus be determined and at the same time the retina can be observed.

2. An ophthalmological appliance according to claim 1, characterised in that the pressure absorption element (25, 32) is transparent and is disposed in the central region of the surface (13) of the contact pressure body (1) which is to be pressed against the cornea (11).

3. An ophthalmological appliance according to claim 1 or 2, wherein the surface (13) of the contact pressure body (1) for pressing against the cornea (11) has a concave shape which is approximately matched to that of the eye (6), characterised in that the face of the pressure absorption element (25, 32) to be placed against the cornea (11) is flat, so that it can be pressed tangentially against the cornea (11).

4. An ophthalmological appliance according to any one of claims 1 to 3, characterised in that the pressure absorption element (25, 32) blanks off a cavity (19) in the contact pressure body (1), which cavity is completely filled with a transparent liquid (26), wherein the refractive index of the liquid (26) is at least approximately the same as that of the contact pressure body (1), and that the liquid (26) acts upon a first pressure sensor (23).

5. An ophthalmological appliance according to claim 4, characterised in that a movable displacement body (30) projects into the cavity (19, 31) filled with liquid (26), at the edge of the appliance which is not utilised optically.

6. An ophthalmological appliance according to any one of claims 1 to 3, characterised in that the pressure absorption element (32) acts directly on at least one pressure sensor (38).

7. An ophthalmological appliance according to claims 4 to 6, characterised in that the pressure sensor (23) is disposed at the edge of the contact pressure body (1) which is not utilised optically.

8. An ophthalmological appliance according to claim 4, characterised in that the pressure absorption element (25) is a transparent diaphragm (25) which blanks off the cavity (19) to provide a liquid-tight seal.

9. An ophthalmological appliance according to any one of claims 4 to 8, wherein the surface (13) of the contact pressure body (1) for pressing against the cornea (11) has a concave shape which approximately matches that of the eye (6), characterised in that at least one second pressure sensor (27) is disposed within the concave face (13) at the edge which is not utilised optically, for measuring the contact pressure of the contact pressure body (1) against the eye (6), and that the pressure measuring device (19, 21, 23, 25, 26, 27) has an electronic evaluation circuit (28) which calculates the difference in the electrical signals from the first (23) and the second (27) sensor, or the difference between the signal from the first sensor (23) and the mean value of the signal from the second sensors (27), wherein an indicating device (33, 39) is provided which displays this difference directly and/or the pressure of the eye determined therefrom.

10. An ophthalmological appliance according to claim 9, characterised in that at least three second sensors (27) are disposed within the concave face (13) at the edge, with angular separations which are at least approximately equal, and that by means of the electronic evaluation circuit (28) the indicator device indicates that the contact pressure body (1) is centred in relation to the eye (6) when the signals from these three pressure sensors (27) agree with each other to a sufficient extent, and indicates the direction or coordinate directions in which the contact pressure body (1) must be moved to reach its position which is centred in relation to the eye (6) when there are different signals from these three sensors (27).

## Revendications

1. Appareil ophtalmologique présentant un corps de pression (1) devant être pressé contre la cornée (11) de l'oeil (6), réalisé en tant qu'élément structurel d'une optique de formation d'images et par l'intermédiaire duquel le fond de l'oeil (7) peut être éclairé et exploré, caractérisé par un dispositif (19, 21, 23, 25, 26, 27) de mesure de pression, destiné à déterminer la pression oculaire interne et comportant au moins un élément (25, 32) absorbant la pression et/ou un capteur de pression (27), respectivement situé sur la surface (13) du corps de pression (1) devant être pressée contre la cornée (11) de l'oeil (6), de telle sorte que la pression oculaire interne puisse ainsi être déterminée et que le fond de l'oeil puisse être simultanément exploré.

2. Appareil ophtalmologique selon la revendication 1, caractérisé par le fait que l'élément (25, 32) absorbant la pression est transparent et se trouve dans la région centrale de la surface (13) du corps de pression (1) devant être pressée contre la cornée (11) de l'oeil.

3. Appareil ophtalmologique selon la revendication 1 ou 2, dans lequel la surface (13) du corps de pression (1), devant être pressée contre la cornée (11) de l'oeil, possède une forme concave approximativement adaptée à l'oeil (6), caractérisé par le fait que la surface de l'élément (25, 32) absorbant la pression, qui doit être appliquée contre la cornée (11), est plane, de façon à pouvoir être pressée tangentiellement contre la cornée (11).

4. Appareil ophtalmologique selon l'une des revendications 1 à 3, caractérisé par le fait que l'élément (25, 32) absorbant la pression obture, dans le corps de pression (1), une cavité (19) intégralement emplie par un fluide translucide (26), l'indice de réfraction du fluide (26) étant au moins approximativement identique à celui du corps de pression (1) et que le fluide (26) agit sur un premier capteur de pression (23).

5. Appareil ophtalmologique selon la revendication 4, caractérisé par le fait que, sur son bord dépourvu d'utilité optique, un corps de refoulement coulissant (30) s'engage dans la cavité (19, 31) remplie de fluide (26).

6. Appareil ophtalmologique selon l'une des revendications 1 à 3, caractérisé par le fait que l'élément (32) absorbant la pression agit directement sur au moins un capteur de pression (38).

7. Appareil ophtalmologique selon les revendications 4 à 6, caractérisé par le fait que le capteur de pression (23) est implanté sur le bord du corps de pression (1) qui est dépourvu d'utilité optique.

8. Appareil ophtalmologique selon la revendication 4, caractérisé par le fait que l'élément (25) absorbant la pression est une membrane transparente (25) qui obture la cavité (19) avec étanchéité aux fluides.

9. Appareil ophtalmologique selon l'une des revendications 4 à 8, dans lequel la surface (13) du corps de pression (1), qui doit être pressée contre la cornée (11) de l'oeil, possède une forme concave approximativement adaptée à l'oeil (6), caractérisé par le fait qu'au moins un second capteur de pression (27) est implanté sur le bord dépourvu d'utilité optique dans la surface concave (13), en vue de mesurer la pression de contact du corps de pression (1) contre l'oeil (6) et que le dispositif (19, 21, 23, 25, 26, 27) de mesure de pression comporte un circuit électronique d'interprétation (28) qui calcule la différence entre les signaux électriques des premier (23) et second (27) capteurs, ou bien la différence entre le signal du premier capteur (23) et la valeur moyenne des signaux des seconds capteurs (27), un dispositif d'affichage (33, 39) étant prévu pour afficher directement cette différence et/ou la pression oculaire établie en fonction de cette différence.

10. Appareil ophtalmologique selon la revendication 9, caractérisé par le fait qu'au moins trois seconds capteurs (27) sont implantés marginalement dans la surface concave (13), selon des espacements angulaires mutuels au moins approximativement égaux et que le dispositif d'affichage (33, 39) associé au circuit électronique d'interprétation (28) affiche, lorsque les signaux de ces trois capteurs de pression (27) concordent suffisamment les uns avec les autres, la position du corps de pression (1) centrée par rapport à l'oeil (6) et, lorsque les signaux de ces trois capteurs (27) sont différents, la direction ou les axes de coordonnées dans laquelle ou lesquelles le corps de pression (1) doit être respectivement déplacé jusqu'à sa position centrée par rapport à l'oeil (6).
